# EUROPEAN PATENT APPLICATION

(11) **EP 4 386 773 A1**
(43) Date of publication of application: **19.06.2024**
(21) Application number: 22856158.5
(22) Date of filing: 09.08.2022
(51) Int. Cl.: G16H 20/70, G16H 10/20, G16H 10/40, G16H 50/30, G16H 50/20, A61B 5/00, G10L 15/10, G10L 15/04

(54) **METHOD AND SYSTEM FOR DIAGNOSING DEMENTIA THROUGH STORY-BASED QUESTION-AND-ANSWER TYPE ASSESSMENT AND STORY-BASED VOICE EXTRACTION**

(30) Priority: 09.08.2021 KR 20210104525; 09.08.2021 KR 20210104529
(71) Applicant: Emocog Inc., Seoul 08708 (KR)
(72) Inventor: LEE, Jun Young, Seoul 04427 (KR); NOH, Yoo Hun, Seoul 06346 (KR)
(74) Representative: Zacco Sweden AB
(86) International application number: PCT/KR2022/011811
(87) International publication number: WO 2023/018161

(57) **Abstract**

The present disclosure relates to a dementia diagnosis system using a story-based question-and-answer-type assessment, for quickly and easily diagnosing dementia without using expert personnel, enabling early dementia diagnosis and quick and accurate screening of patients with mild symptoms, and performing a question-and-answer-type assessment through voice, thereby making it possible to easily examine even elderly subjects who have reading difficulties, and the dementia diagnosis system includes a data inputter 10 for inputting an original story, a data storage 20 for storing text data that is input through the data inputter 10, a voice outputter 30 for outputting diagnostic voice data to the outside as a voice, and a voice receiver 40 for receiving a response voice from an examinee, a controller 50 that is connected to various devices including the data inputter 10 to control them, generate an adapted story, a question, a correct answer based on the original story, receives a response from the examinee, and determines dementia by comparing the correct answer with the response.

## Description

### Technical Field

The present disclosure relates to a dementia diagnosis system and method, and more specifically, to a dementia diagnosis system and method using a story-based question-and-answer-type assessment and story-based voice extraction, for quickly and easily diagnosing dementia without using expert personnel, enabling early dementia diagnosis and quick and accurate screening of patients with mild symptoms, and performing a question-and-answer-type assessment through voice or providing a story through voice, thereby making it possible to easily examine even elderly subjects who have reading difficulties.

### Background Art

Dementia generally refers to a series of symptoms caused by brain disease. As dementia progresses, it affects thinking ability, behavior, and performance of daily life, resulting in a decline in cognitive ability and thus a lack of ability for daily activities.

In general, doctors diagnose significant impairment of two or more cognitive functions, as dementia. These cognitive functions include memory, language skills, understanding information, spatial skills, judgment, and attention. People with dementia may have difficulty in solving problems and controlling their emotions, and may experience personality changes.

The exact symptom that a dementia patient experiences depends on which part of the brain is damaged by the disease that caused the dementia. Dementia is when some of nerve cells in the brain stop functioning and lose connection with other cells, leading to death, and this type of dementia usually progresses steadily. That is, dementia gradually spreads to the brain, and the patient's symptoms worsen over time.

Dementia is a representative age-related neurodegenerative brain disease, with a prevalence of approximately 5 % to 10 % in people over 65 years of age worldwide, and most dementia patients have symptoms of progressive cognitive dysfunction, hallucinations, delusions, and loss of ability to live.

In the case of Alzheimer's disease, which is the most representative form of dementia, clumps of waste containing a protein called amyloid beta are observed in nerve cells of the brain cortex and around nerve fiber bundles and brain cells, and this waste is presumed to cause necrosis of nerve cells.

Alzheimer's disease is the most common disease accounting for about 70 % of dementia, and it is recently estimated that about 600,000 people will have Alzheimer's disease annually in Republic of Korea.

Alzheimer's disease, which is a representative degenerative dementia disease, is a devastating disease that causes a deterioration in the cognitive function and difficulties in daily lives due to the degeneration of cerebral nerve cells, and leads to death, causing pain not only to the patient but also to the family caring for the patient.

Diagnosis of dementia is made by identifying impairments in daily life and social activities due to cognitive impairment, through detailed medical history hearing and assessments on symptoms, and confirming dementia by examining cerebrovascular disease and brain atrophy through brain imaging diagnosis.

In an early stage of dementia, it is difficult to distinguish dementia from senescent forgetfulness, and thus, dementia is diagnosed by performing a neuropsychological test for comprehensively evaluating not only memory, but also language ability, calculation ability, spatiotemporal perception ability, judgment, and the like. In order to diagnose dementia, it is necessary to perform additional detailed tests based on various pieces of information obtained through interviews with patients/guardians and screening tests. The additional detailed tests include neuropsychological tests (e.g., Seoul Neuropsychological Screening Battery (SNSB)), blood tests, and various types of brain imaging (e.g., computed tomography (CT), magnetic resonance imaging (MRI), or positron emission tomography (PET)).

As an example of the additional detailed test, MRI may play an important role in identifying types of dementia. This test may identify whether a case of dementia is close to Alzheimer's disease dementia or vascular dementia, and may also be of some help in determining whether the case of dementia is caused by another disease.

However, the above related art has the following issues.

Because dementia such as Alzheimer's disease is irreversible and no cure has been developed, the best way is to diagnose dementia early and slow its progression. However, diagnosis of dementia through detailed medical history hearing and assessments by professional doctors cannot be performed quickly on numerous patients due to a lack of professional manpower, and increases the cost of screening dementia patients from among the entire population at the national level.

In addition, related-art brain imaging used for diagnosing dementia requires expensive and precise equipment, however, enables confirmation of dementia only when a brain disease or brain atrophy is in a significantly advanced state, and thus may be unsuitable for early diagnosis of dementia.

In addition, dementia patients are usually elderly, illiterate, less educated, and have difficulty in recognizing letters, resulting in difficulty in an examination using questionnaires, and thus, an examination requires a lot of time and manpower.

Thus, there is an urgent need to develop appropriate and groundbreaking techniques for easily and quickly diagnosing dementia at an early stage.

### Disclosure

### Technical Problem

The present disclosure is devised to solve the above issues of the related art, and provides
a dementia diagnosis system and method using a story-based question-and-answer-type assessment, for quickly and easily diagnosing dementia without using expert personnel, enabling early dementia diagnosis and quick and accurate screening of patients with mild symptoms, and performing a question-and-answer-type assessment through voice, thereby making it possible to easily examine even elderly subjects who have reading difficulties.

### Technical Solution

In order to achieve the above objectives, a "dementia diagnosis system using a story-based question-and-answer assessment" according to the present disclosure includes: a data inputter configured to input text data regarding an original story; a data storage configured to store the text data that is input through the data inputter, and dementia information for each examinee; a voice outputter configured to output diagnostic voice data to an outside to allow an examinee to listen to the diagnostic voice data; a voice receiver configured to receive a response spoken by the examinee; and a controller that is connected to the data inputter, the data storage, the voice outputter, and the voice receiver, and comprises a story data generation unit configured to automatically generate adapted story data, question data, and correct answer data based on an original story that is input as text data, a response data generation unit configured to receive a response from the examinee and generate response data, a dementia determination unit configured to compare and analyze the correct answer data with the response data to identify an incorrect response rate, and determine occurrence of dementia and/or dementia status according to the incorrect response rate, the controller being configured to control the data storage, the voice outputter, and the voice receiver to sequentially output the adapted story data and the question data, receive responses from the examinee to output questions, and store identified dementia information for each examinee, wherein the original story is in a form of a story centered around a character that the examinee already knows, and an event related to the character, and consists of a plurality of sentences, the adapted story data is data obtained by extracting some of noun-type words included in the original story, changing the extracted noun-type words to other noun-type words of a same or similar type to generate an adapted story, and converting the adapted story into voice data, the question data is data obtained by generating a plurality of questions related to the adapted story by using the changed noun-type words included in the adapted story as correct answers, and converting the questions into voice data, the correct answer data is data obtained by extracting the correct answers corresponding to the questions and converting the extracted correct answers into voice data, and the response data is voice data obtained by receiving and storing responses from the examinee to the questions through the voice receiver.

In addition, a "dementia diagnosis system using story-based voice extraction" according to the present disclosure includes: a data storage for storing diagnostic voice data including story data for dementia diagnosis, patient voice feature data extracted by comparing and analyzing voice features of normal people with voice features of dementia patients, and dementia information for each examinee; a voice output device configured to output the diagnostic voice data stored in the data storage to the outside to allow the examinee to listen to the diagnostic voice data; a voice receiver configured to receive a voice spoken by the examinee and convert the voice into voice data; and a controller that is connected to the data storage, the voice outputter, and the voice receiver, is configured to control the voice receiver, the data storage, and the voice outputter to receive a voice of the examinee after outputting the story data, and includes an utterance request unit configured to request the examinee to recall and speak the content of an output story after the output of the story data is completed, a voice feature analysis unit configured to analyze voice features in voice data received from the examinee, and a dementia determination unit configured to determine occurrence of dementia and/or dementia status by comparing the voice features of the examinee analyzed through the voice feature analysis unit, with the patient voice feature data, the controller being further configured to perform control to store, in the data storage, dementia information for each examinee determined by the dementia determination unit, wherein the story data is obtained by generating an original story that includes a plurality of sentences and is in the form of a story centered around a character that the examinee already knows and an event related to the character, extracting some of noun-type words included in the original story, changing the extracted noun-type words to other noun-type words of the same or similar type to generate an adapted story, and converting the adapted story into voice data.

### Advantageous Effects

According to the present disclosure, it is possible to quickly and easily diagnose dementia without using expert personnel, enabling early dementia diagnosis and quick and accurate screening of patients with mild symptoms, and performing a question-and-answer-type assessment through voice or providing a story through voice, thereby making it possible to easily examine even elderly subjects who have reading difficulties, and thus, enabling screening of hundreds of thousands of dementia patients among the entire population at a national level to be performed quickly and easily at a relatively low cost.

### Description of Drawings

FIG. 1 is a schematic diagram illustrating a dementia diagnosis system using a story-based question-and-answer assessment, according to the present disclosure.
FIG. 2 is a schematic configuration diagram illustrating a controller of a dementia diagnosis system using a story-based question-and-answer assessment, according to the present disclosure.
FIG. 3 is an exemplary diagram illustrating an example of an original story used by a dementia diagnosis system according to the present disclosure.
FIG. 4 is an exemplary diagram illustrating an example of an adapted story used by a dementia diagnosis system according to the present disclosure.
FIG. 5 is an exemplary diagram illustrating an example of a story notification text used by a dementia diagnosis system according to the present disclosure.
FIG. 6 is an exemplary diagram illustrating an example of a question notification text used by a dementia diagnosis system according to the present disclosure.
FIG. 7 is an exemplary diagram illustrating examples of questions used by a dementia diagnosis system according to the present disclosure;
FIG. 8 is a schematic configuration diagram illustrating a dementia diagnosis system according to another embodiment of the present disclosure.
FIG. 9 is a schematic configuration diagram illustrating a controller of a dementia diagnosis system according to another embodiment of the present disclosure.
FIG. 10 is a flowchart of a dementia diagnosis method using a story-based question-and-answer assessment, according to the present disclosure.
FIG. 11 is a schematic configuration diagram illustrating a dementia diagnosis system through story-based voice extraction, according to the present disclosure.
FIG. 12 is a schematic configuration diagram illustrating a controller of a dementia diagnosis system through story-based voice extraction, according to the present disclosure.
FIG. 13 is a flowchart of a dementia diagnosis method through story-based voice extraction, according to the present disclosure.

### Best Mode

The present disclosure relates to a dementia diagnosis system using a story-based question-and-answer-type assessment, for quickly and easily diagnosing dementia without using expert personnel, enabling early dementia diagnosis and quick and accurate screening of patients with mild symptoms, and performing a question-and-answer-type assessment through voice, thereby making it possible to easily examine even elderly subjects who have reading difficulties, and the dementia diagnosis system includes a data inputter 10 for inputting an original story, a data storage 20 for storing text data that is input through the data inputter 10, a voice outputter 30 for outputting diagnostic voice data to the outside as a voice, and a voice receiver 40 for receiving a response voice from an examinee, a controller 50 that is connected to various devices including the data inputter 10 to control them, generate an adapted story, a question, a correct answer based on the original story, receives a response from the examinee, and determines dementia by comparing the correct answer with the response.

### Mode for Invention

Hereinafter, embodiments of the present disclosure will be described in more detail with reference to the accompanying drawings. The present disclosure may, however, be embodied in many different forms and should not be construed as being limited to the embodiments set forth herein.

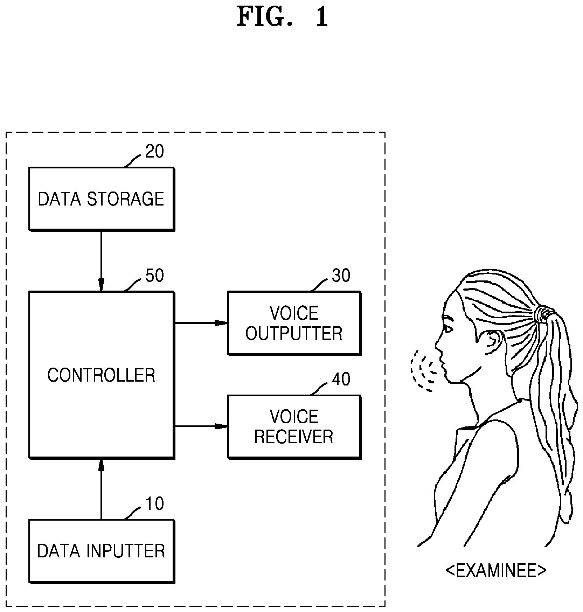

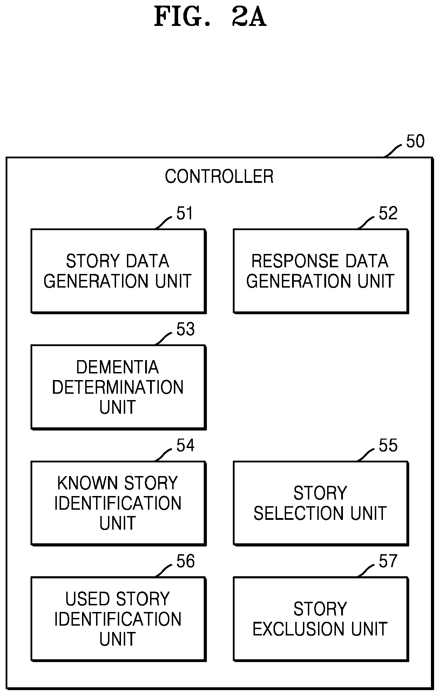

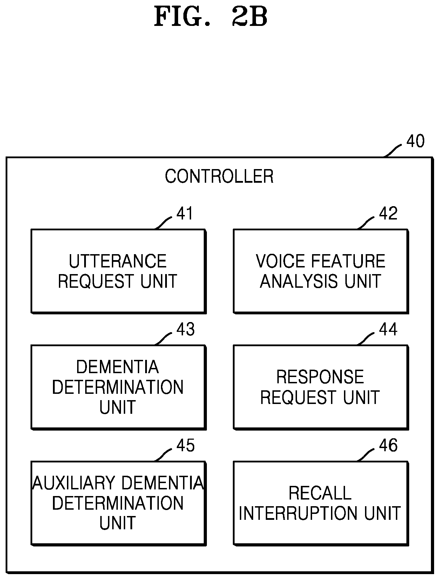

FIGS. 1 to 7 are diagrams illustrating a dementia diagnosis system according to the present disclosure. As illustrated in the drawings, a dementia diagnosis system using a story-based question-and-answer assessment according to the present disclosure includes a data inputter 10 for inputting an original story, a data storage 20 for storing text data that is input through the data inputter 10, a voice outputter 30 for outputting diagnostic voice data to the outside as a voice, and a voice receiver 40 for receiving a response voice from an examinee, a controller 50 that is connected to various devices including the data inputter 10 to control them, generate an adapted story, a question, a correct answer based on the original story, receives a response from the examinee, and determines dementia by comparing the correct answer with the response.

The data inputter 10 is an input device capable of inputting ordinary data into a storage device, and allows a user to input text data regarding an original story into the data storage 20 through the controller 50.

Here, the original story is in the form of a character centered around a person who is already known to the examinee, and an event related to the character, consists of a plurality of sentences, and is written and prepared by a producer or an operator.

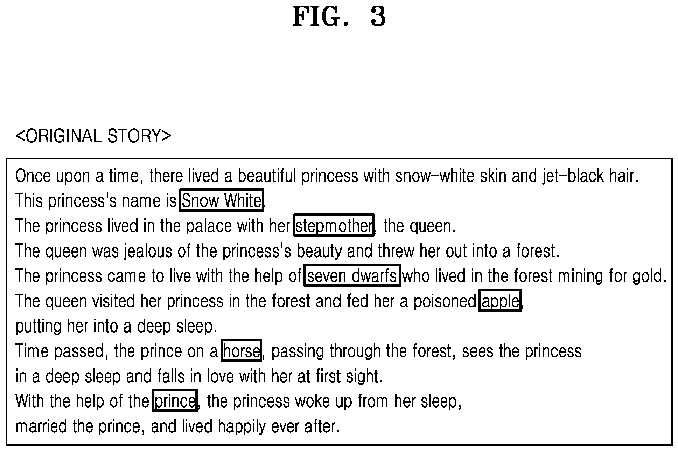

It is preferable that the original story is a fairy tale or a fable that the examinee already knows. For example, the original story may be a fairy tale such as Snow White illustrated in FIG. 3, or a fable such as Aesop's Fables, and a plot summarized in 15 sentences or less may be used as the original story.

Here, that the examinee already knows the original story means that the examinee has already known the plot for a long time, such as a classic, traditional, or widely known fairy tale or fable, and is able to easily recall it from memory.

It is preferable to prepare a plurality of such original stories such that various fairy tales or fables may be used, and this is to increase accuracy by using different stories when repeatedly examining the same person, and to appropriately select a fairy tale or fable known to the examinee and use it for diagnosis.

The data storage 20 is a storage device capable of storing digital data, such as a memory or a hard disk, and is for storing all data including text data input through the data inputter 10, and dementia information for each examinee.

The voice outputter 30 is a unit capable of outputting digital data as a voice, such as a speaker, and outputs diagnostic voice data to the outside such that the examinee may listen to the voice. Here, the diagnostic voice data refers to voice data regarding an adapted story and a question that are output for determining dementia.

The voice receiver 40 is a unit for receiving a voice, such as a microphone, and serves to receive a response spoken by the examinee.

The controller 50 is connected to the data inputter 10, the data storage 20, the voice outputter 30, and the voice receiver 40, and includes a story data generation unit 51 for automatically generating adapted story data, question data, and correct answer data based on an original story that is input as text data, a response data generation unit 52 for receiving a response from the examinee and generating response data, and a dementia determination unit 53 for comparing and analyzing the correct answer data with the response data to identify an incorrect response rate, and determining occurrence of dementia and/or dementia status according to the incorrect response rate.

The controller 50 serves to control the data storage 20, the voice outputter 30, and the voice receiver 40 to sequentially output the adapted story data and the question data, receive a response of the examinee to an output question, and store identified dementia information for each examinee.

The story data generation unit 51 serves to automatically generate adapted story data, question data, and correct answer data based on text data regarding an input original story.

Here, the adapted story data refers to data obtained by extracting some of noun-type words included in the original story, changing the extracted noun-type words to other noun-type words of the same or similar type to generate an adapted story, and converting the adapted story into voice data.

That is, the adapted story is generated by extracting some of the noun-type words included in the original story and changing the extracted noun-type words into other words of a similar type.

For example, as illustrated in FIG. 3, noun-type words extracted from an original story are marked with red quadrangles, and the extracted noun-type words marked with the red quadrangles in FIG. 3 are 'Snow White', 'stepmother', 'seven', 'dwarfs', 'apple', 'horse', and 'prince'.

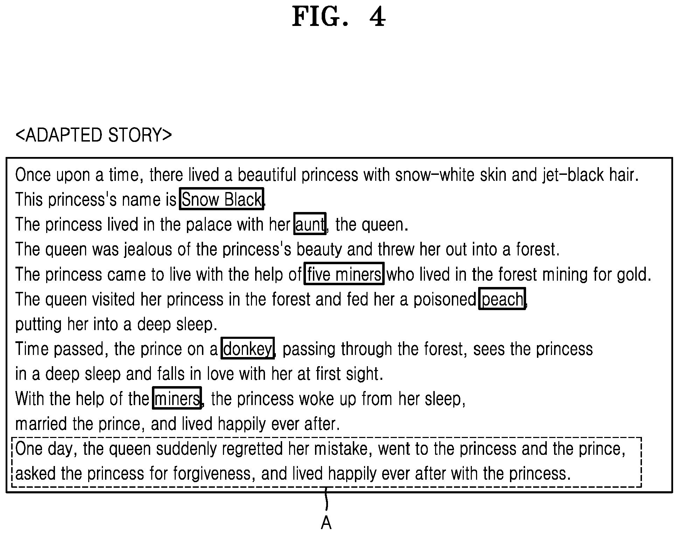

As illustrated in FIG. 4, the noun-type words extracted from the original story are changed to other words of similar types, such as 'Snow Black', 'aunt', 'five', 'miners', 'peach', 'donkey', and 'miners'.

That is, 'Snow White' in the original story is changed to 'Snow Black', 'stepmother' in the original story is changed to 'aunt', 'seven' in the original story is changed to 'five', 'dwarfs' in the original story is changed to 'miners', 'apple' in the original story is changed to 'peach', 'horse' in the original story is changed to 'donkey', and 'prince' in the original story is changed to 'miners'.

As such, by changing some of the noun-type words of the original story illustrated in FIG. 3, the adapted story as illustrated in FIG. 4 is generated.

It is preferable that such an adapted story consists of 5 to 15 sentences and contains 20 to 50 different noun-type words, and this is because when the number of sentences in the adapted story is less than 5 or the number of noun-type words is less than 20, the adapted story is too short and thus easily recalled, and thus, the short-term memory of the examinee cannot be effectively tested or dementia patients cannot be properly screened.

In addition, when the number of sentences in the adapted story is greater than 15 or the number of noun-type words is greater than 50, the adapted story is too long and thus difficult to recall, and thus, there is a risk that even a normal person may be misdiagnosed as a dementia patient.

It is preferable that the adapted story includes at least one additional sentence that is artificially generated to be different from the content of the original story but associated with and connected to the content of the adapted story.

An example of the additional sentence is indicated by A in FIG. 4, and it serves to cause confusion in and thus interrupt a recall by the examinee. That is, the examinee, especially a dementia patient, is unable to recall the entire content of the adapted story due to the recall interruption caused by the additional sentence, and as a result, screening of dementia patients is performed more accurately.

Here, that the additional sentence is different from the content of the original story means that the content of the additional sentence is not in the content of the original story, and that the additional sentence is associated with and connected to the content of the adapted story means the content of the additional sentence is connected to the development of the adapted story. The additional sentence may be generated directly by a producer and then input through the data inputter 10, or may be automatically generated by the controller 50 with stimulating content that includes the main character.

In addition, it is preferable that the additional sentence is placed at the end of the adapted story as illustrated in FIG. 4, which is to effectively interrupt a recall of the adapted story by the examinee.

The question data is data obtained by generating a plurality of questions related to the adapted story by using the changed noun-type words included in the adapted story as correct answers, and converting the questions into voice data.

Here, it is preferable that the number of questions is 3 to 12, and this is because, when the number of questions is less than 3, the discrimination performance may be poor, and thus, the memory of the examinee may not be properly tested, and when the number of questions is greater than 12, it is beyond the short-term memory of the examinee, and thus, a memory test may not be performed accurately.

In addition, it is preferable that the questions are about whether an event, an action, a situation, and content presented in the adapted story are correct, and this is to appropriately test the short-term memory through simple and plain fact-oriented questions.

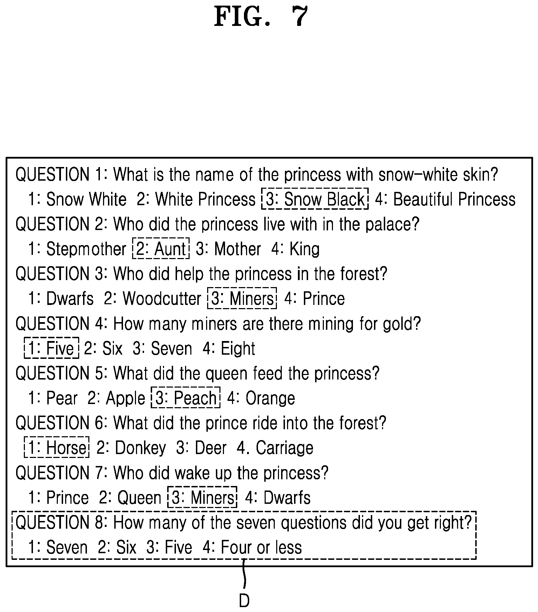

In addition, it is preferable that the question is a multiple-choice type in which incorrect answers include noun-type words included in the original story of the same or similar type as the correct answer as illustrated in FIG. 7. For example, in a four-choice question as illustrated in FIG. 7, noun-type words included in the original story, i.e., 'Snow White', 'stepmother', 'seven', 'dwarfs', 'apple', 'horse', and 'prince' are included as incorrect answers among the four choices, causing confusion in a recall by the examinee, such that the examinee selects the content of the fairy tale that the examinee knows, i.e., a wrong answer, and thus, the examinee may be properly identified as a dementia patient.

That is, some examinees cannot recall the content of the adapted story that contains the correct answer to the question, but recall the content of the original story that they have known for a long time, and then select an answer based on the original story, which results in an incorrect answer, and such examinees who select incorrect answers are diagnosed and selected as dementia patients.

In addition, it is preferable that the last question of the questions is for asking the examinee about his/her correct answer rate. The question for asking the examinee about his/her correct answer rate is indicated by D in FIG. 7 and is for allowing an operator to evaluate the metacognitive ability of the examinee to recognize what he/she knows and does not know. This metacognitive ability assessment may be used as data for confirming once again the accuracy of dementia diagnosis by the present system.

The correct answer data is data obtained by extracting the correct answer corresponding to the question and converting the extracted correct answer into voice data. The correct answer data is compared with the response data collected from the examinee, and used as data for identifying the incorrect response rate.

Here, the correct answer refers to a noun-type word extracted from the original story and changed in the adapted story, and for example, in the adapted story of FIG. 4, 'Snow Black', 'aunt', 'five', 'miners', 'peach', 'donkey', and 'miners', which are marked with red boxes, correspond to correct answers.

The response data generation unit 52 serves to automatically generate response data by receiving an answer of the examinee as a voice. Here, the response data is voice data obtained by receiving and storing a response from the examinee to the question through the voice receiver 40.

The dementia determination unit 53 serves to compare the generated correct answer data with the response data to determine occurrence of dementia and/or dementia status through the incorrect response rate for the question.

That is, when asked about 'Snow White' in the adapted story, the examinee is confused with the original story of 'Snow White' that he/she knows, and thus selects an incorrect answer, and when the number of incorrect answers is large and thus the incorrect answer rate is high, the examinee is determined as a dementia patient.

The dementia determination unit 53 compares and analyzes a voice of the correct answer data with the voice of the response data to confirm the correct answer and an incorrect answer, this is to ensure that an incorrect answer is more accurately determined through voice recognition and voice comparison.

Here, in FIG. 7, correct answers to the questions are indicated by red boxes, other choices than the correct answers are naturally treated as incorrect answers, the correct answer to 'Question 8' is confirmed by evaluating 'Question 1' to 'Question 7', and the incorrect answer rate is the proportion of incorrect answers for all questions.

It is preferable that the dementia determination unit 53 determines that the examinee has dementia when the incorrect response rate is greater than at least 25 %, determines that the examinee has mild dementia when the incorrect response rate is 25 % to 40 %, and determines that the examinee has severe dementia when the incorrect response rate is greater than at least 40 %, and these percentages are determined through comparative analysis and confirmation of incorrect response rates of examinees belonging to a normal group and examinees belonging to a dementia group through clinical experiments with a plurality of examinees.

That is, the examinees with incorrect response rates greater than 25 % are all identified as dementia patients, the examinees with incorrect response rates between 25 % and 40 % are classified as mild dementia patients, and the examinees with incorrect response rates greater than 40 % are classified as severe dementia patients.

The controller 50 receives a story notification text for notifying that the adapted story will be output and then a question related to the adapted story will be presented, converts the story notification text into voice data, and controls the voice outputter 30 to output the story notification text first before outputting the adapted story.

The story notification text is produced and input as text data by the producer or operator, and is converted into voice data and then output.

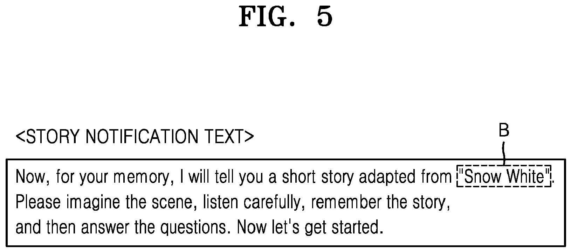

An example of the story notification text is illustrated in FIG. 5, and the output of the story notification text serves to inform the examinee in advance that the adapted story will be output as a voice, a question will be presented, and the examinee will need to give an answer.

It is preferable that a sentence containing the title of the fairy tale or fable presented as the adapted story is placed at the beginning of the story notification text. The title of the fable or fairy tale is presented as indicated by B in FIG. 5 ("Snow White"), and the title of the fairy tale to be output as a voice, for example, "Snow White", is provided to allow the examinee to recall the related fairy tale "Snow White" in advance such that the examinee may easily recall the entire plot of "Snow White".

The controller 50 receives a question notification text for notifying that a preset number of questions will be presented, converts the question notification text into voice data, and controls the voice outputter 30 to output the question notification text after completion of the output of the adapted story.

The question notification text is produced and input as text data by the producer or operator, converted into voice data, and then output.

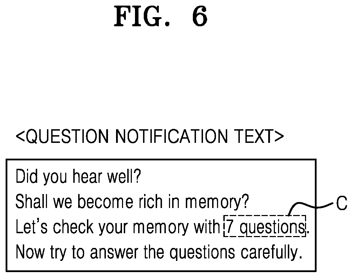

The output of the question notification text serves to notify that a question will be output immediately after the adapted story is output, and the examinee will need to answer the question, and notify of the number of questions in advance as illustrated in C in FIG. 6 ("7 questions") such that the examinee may appropriately respond to output questions.

In the dementia diagnosis system, a plurality of original stories may be prepared and input respectively to generate a plurality of adapted stories, and questions and correct answers related to each adapted story may be generated, which is for more accurate diagnosis and determination of dementia through various question-and-answer assessments with a plurality of original stories.

The controller 50 further includes a known story identification unit 54 for, in a state where a plurality of original stories are input as described above, outputting a story identification text as a voice to the examinee to identify which story the examinee already knows from among a plurality of adapted stories before an adapted story is output, and a story selection unit 55 for selecting an adapted story that is identified to be already known by the examinee such that the adapted story identified through the known story identification unit 54 is output.

For example, the known story identification unit 54 outputs a story identification text containing the titles of the plurality of adapted stories (e.g., 'Snow White', 'Cinderella', 'The Little Mermaid', 'Pinocchio', and 'The Prince and the Pauper') as a voice, to allow the examinee to check fairy tales or fables stored in a diagnostic device as adapted stories.

The story selection unit 55 allows the examinee to listen to the story identification text and select an adapted story that the examinee already knows. For example, adapted stories of 'Snow White', 'Cinderella', 'The Little Mermaid', 'Pinocchio', and 'The Prince and the Pauper' are stored, the titles of these stories are output, and then when the examinee already knows 'Cinderella', the examinee may select 'Cinderella' by answering with a voice or manipulating a button.

When 'Cinderella' is selected, an adapted story and questions related to 'Cinderella' are output to the examinee for dementia diagnosis, such that dementia diagnosis is performed based on a fairy tale or fable that the examinee already knows, while appropriately preventing dementia diagnosis with a fairy tale or fable that the examinee does not know, and thus, the accuracy of dementia diagnosis is improved.

The controller 50 further includes a used story identification unit 56 for outputting a voice to the examinee before the adapted story is output, to identify which story among the plurality of adapted stories has been previously used for a diagnostic test, and a story exclusion unit 57 for excluding, from output, an adapted story identified by the used story identification unit 56 to have been previously used.

The used story identification unit 56 serves to identify a story that has been previously used in a diagnostic test of the examinee from among the plurality of adapted stories, and the story exclusion unit 57 serves to exclude the adapted story identified to have been used such that it is not output to the examinee.

As such, by excluding an adapted story that has been used for a diagnostic test of the examinee, a decrease in diagnostic accuracy due to repeated use of the same content and the same question is appropriately prevented.

The dementia diagnosis system configured as described above may be implemented as an integrated dementia diagnosis system in which the data inputter 10, the data storage 20, the voice outputter 30, the voice receiver 40, and the controller 50 are modularized and integrated, and may be implemented as an automatic dementia diagnosis device in which all functions are programmed or automated through artificial intelligence or the like.

As described above, the dementia diagnosis system is a groundbreaking invention that allows quick and accurate screening of dementia by changing key words from an original classic story that an examinee already knows and is familiar with, to new words, presenting a resulting adapted story to cause memory consolidation, and presenting original words of the original classic story as incorrect answers to maximize a memory interference effect.

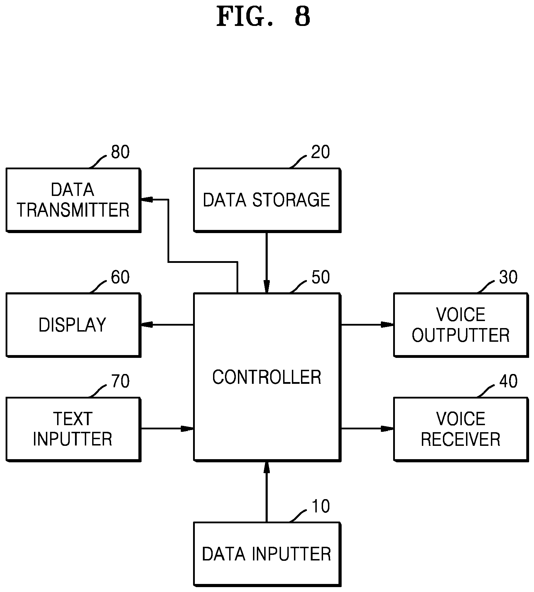

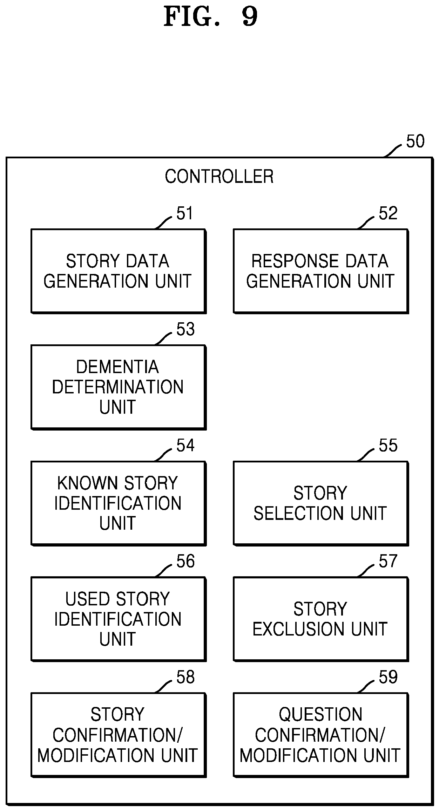

FIGS. 8 and 9 are schematic diagrams illustrating a dementia diagnosis system according to another embodiment of the present disclosure. As illustrated in the drawings, the dementia diagnosis system according to another embodiment of the present disclosure further includes a display 60 connected to the controller 50 and capable of displaying text data on a screen, and a text inputter 70 connected to the controller 50 and capable of modifying text data by inputting a text.

The display 60 is a unit for displaying a text or an image on a screen, such as a monitor, and serves to display the adapted data and question data as text data.

The text inputter 70 is a unit capable of inputting and modifying a text, such as a keyboard, and allows modification of text data of the adapted data and question data.

The controller 50 further includes a story confirmation/modification unit 58 for displaying the adapted story as text data on the display 60 to allow a producer or an operator to confirm the adapted story and modify it through the text inputter 70, and a question confirmation/modification unit 59 for displaying the question on the display 60 as text data to allow the producer or operator to confirm the question and modify it through the text inputter 70.

The story confirmation/modification unit 58 allows a person, such as a producer or an operator, to directly confirm and modify a story that is automatically adapted by the controller 50 so as to generate a high-quality adapted story in which the development of events is more natural and smoother.

The question confirmation/modification unit 59 allows a person, such as a producer or an operator, to directly confirm and modify a question that is automatically generated by the controller 50, so as to generate a natural, smooth, and difficulty-adjusted question.

The dementia diagnosis system further includes a data transmitter 80 connected to the data storage 20 and controlled by the controller 50. The data transmitter 80 is, for example, a wired or wireless transmitting/receiving device, and is controlled by the controller 50 to transmit dementia information for each examinee stored in the data storage 20 to an external device through a transmission line or a network.

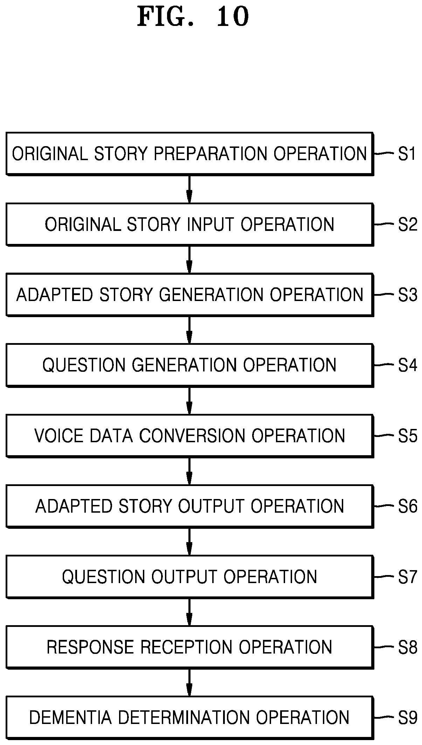

FIG. 10 is a flowchart of a dementia diagnosis method according to the present disclosure.

Referring to FIG. 10, a dementia diagnosis method using a story-based question-and-answer assessment according to the present disclosure includes an original story preparation operation (S1) of preparing an original story, an original story input operation (S2) of converting the original story into text data and inputting it into a diagnostic device, an adapted story generation operation (S3) of generating an adapted story by adapting the input original story, a question generation operation (S4) of generating a plurality of questions with changed noun-type words included in the adapted story as correct answers, a voice data conversion operation (S5) of converting the adapted story and the questions into voice data, an adapted story output operation (S6) of outputting the voice data of the adapted story to the outside, a question output operation (S7) of sequentially outputting the voice data of the questions to the outside, a response reception operation (S8) of receiving responses from an examinee to the questions, and a dementia determination operation (S9) of determining occurrence of dementia through an incorrect response rate on the questions and the responses, and these operations are sequentially performed to diagnose dementia.

In the question generation operation (S4), the questions include 3 to 12 questions, are about whether events, actions, situations, and content presented in the adapted story are correct, and are of a multiple-choice type in which incorrect answers include noun-type words included in the original story of the same or similar type as correct answers, and a question for asking the examinee about his/her correct answer rate may be placed as the last question.

In the original story preparation operation (S1), the original story may be a fairy tale or a fable that the examinee already knows.

In the adapted story generation operation (S3), the adapted story may include 5 to 15 sentences and contain 20 to 50 different noun-type words, and the adapted story generation operation (S3) may further include a sentence addition process of adding at least one artificially generated additional sentence that is different from the content of the original story and is associated with and connected to the content of the adapted story, and the additional sentence may be placed at the end of the adapted story.

The adapted story output operation (S6) may further include a story notification output process of, before outputting the adapted story, outputting, as a voice, a story notification text for notifying that the adapted story will be output and then a question related to the adapted story will be presented after the output of the adapted story, and the adapted story may be a fairy tale or a fable that the examinee already knows, and the story notification output process may further include a process of outputting a sentence including the title of the fairy tale or fable presented as the adapted story, at the beginning of the story notification text.

The adapted story output operation (S6) may further include a question notification output process of outputting, as a voice, a question notification text for notifying that a preset number of questions will be presented after the output of the adapted story is completed.

In the dementia determination operation (S9), the examinee may be determined as a dementia patient when the incorrect response rate is greater than at least 25 %, as a mild dementia patient when the incorrect response rate is 25 % to 40 %, and as a severe dementia patient when the incorrect response rate is greater than at least 40 %.

The original story may include a plurality of original stories, and be input to generate a plurality of adapted stories, and questions related to each adapted story may generated, and the adapted story output operation (S6) may further include a known story identification process of outputting, as a voice, a story identification text to the examinee for identifying which story the examinee already knows from among the plurality of adapted stories before the adapted story is output, and a story selection process of selecting an adapted story that is identified to be already known by the examinee such that the adapted story identified through the known story identification process is output.

The original story may include a plurality of original stories, and be input to generate a plurality of adapted stories, and questions related to each adapted story may generated, and the adapted story output operation (S6) may further include a used story identification process of, before the adapted story is output, asking the examinee which of the plurality of adapted stories has been previously used for a diagnostic test, and a story exclusion process of excluding, from output, an adapted story that is identified through the used story identification process to have been previously used.

The adapted story generation operation (S3) may further include a story confirmation/modification process of displaying the adapted story generated as text data on a display such that a producer or an operator may confirm and modify the adapted story.

The question generation operation (S4) may further include a question confirmation/modification process of displaying the questions generated as text data on a display such that a producer or an operator may confirm and modify the questions.

The data transmitter 80 may transmit dementia information for each examinee, and thus, information about diagnosed and screened dementia patients may be easily provided to management servers of national and private organizations for dementia management and screening.

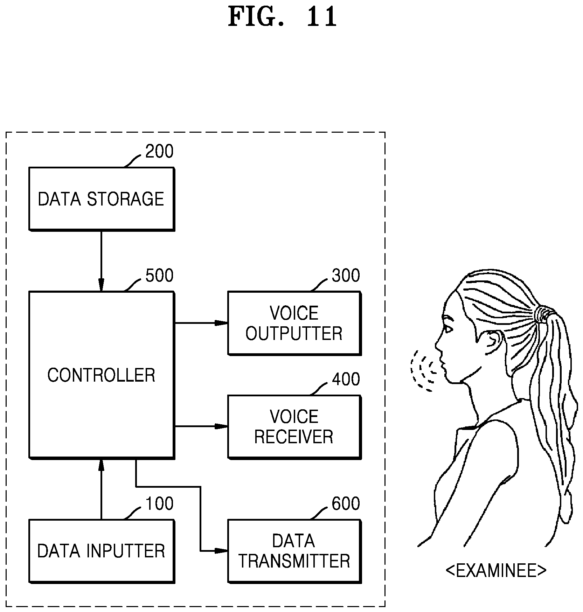

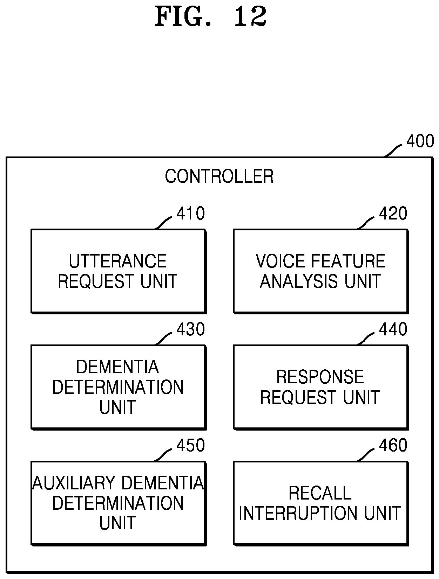

FIGS. 11 and 12 are diagrams illustrating a dementia diagnosis system according to the present disclosure. As illustrated in the drawings, a dementia diagnosis system using story-based voice extraction according to the present disclosure includes a data storage 100 for storing diagnostic voice data for dementia diagnosis and patient voice feature data, a voice outputter 200 for outputting the diagnostic voice data stored in the data storage 100 as a voice to the outside, a voice receiver 300 for receiving a voice of an examinee, and a controller 400 that is connected to the data storage 100, the voice outputter 200, and the voice receiver 300 to control them, and determines dementia by comparing a voice feature of the examinee with the patient voice feature data.

The data storage 100 may be a unit capable of storing digital data, such as a memory or a hard disk, and may store diagnostic voice data including story data for dementia diagnosis, patient voice feature data including voice features of dementia patients extracted by comparing and analyzing voice features of normal people with voice features of the dementia patients, and dementia information for each examinee.

Here, the patient voice feature data is distinguished from the voice features of the normal people, and is obtained by converting, into data, common voice features extracted from voices of a plurality of dementia patients.

The voice features of the normal people and dementia patients include one or more of the fundamental frequency, utterance rate, pause, shimmer, jitter, formant, and voice spectrum of a voice.

The fundamental frequency of the voice refers to a frequency representing the average and habitual sound level of the voice, and the utterance rate refers to a speech rate and an articulation rate and represents the speed at which a person speaks.

The shimmer (shimmer0) numerically represents the regularity of voice amplitude changes, the jitter (jitter0) numerically represents the change rate of vocal fold vibration, the formant (formant0) represents a frequency component that characterizes a vowel, and the voice spectrum represents the distribution of voice by frequency.

In addition, the story data is obtained by generating an original story that includes a plurality of sentences and is in the form of a story centered around a character that the examinee already knows and an event related to the character, extracting some of noun-type words included in the original story, changing the extracted noun-type words to other noun-type words of the same or similar type to generate an adapted story, and converting the adapted story into voice data.

The story data is obtained by converting the adapted story into voice data, and is for easily outputting and providing the adapted story to the examinee as a voice at a later stage.

It is preferable that the original story is a fairy tale or a fable that the examinee already knows. For example, the original story may be a fairy tale such as Snow White illustrated in FIG. 3, or a fable such as Aesop's Fables, and a plot summarized in 15 sentences or less may be used as the original story.

Here, that the examinee already knows the original story means that the examinee has already known the plot for a long time, such as a classic, traditional, or widely known fairy tale or fable, and is able to easily recall it from memory.

The adapted story is an adaptation of the original story and is obtained by extracting some of noun-type words included in the original story and changing the extracted noun-type words to other words of a similar type. For example, as illustrated in FIG. 3, noun-type words extracted from an original story are marked with red quadrangles. The extracted noun-type words marked with the red quadrangles are 'Snow White', 'stepmother', 'seven', 'dwarfs', 'apple', 'horse', and 'prince'.

As illustrated in FIG. 4, the noun-type words extracted from the original story are changed to other words of similar types, such as 'Snow Black', 'aunt', 'five', 'miners', 'peach', 'donkey', and 'miners'.

That is, 'Snow White' in the original story is changed to 'Snow Black', 'stepmother' in the original story is changed to 'aunt', 'seven' in the original story is changed to 'five', 'dwarfs' in the original story is changed to 'miners', 'apple' in the original story is changed to 'peach', 'horse' in the original story is changed to 'donkey', and 'prince' in the original story is changed to 'miners'.

When changing a word to a similar word as described above, the word may be changed automatically by extracting the similar word, or a producer or an operator may directly intervene to change the word. Here, it is preferable that the producer or operator directly changes the words or intervene in changing of some words to make the adaptation natural.

By changing some noun-type words presented in the original story, an adapted story as illustrated in FIG. 4 is completed.

It is preferable that such an adapted story consists of 5 to 15 sentences and contains 20 to 50 different noun-type words, and this is because when the number of sentences in the adapted story is less than 5 or the number of noun-type words is less than 20, the adapted story is too short and thus easily recalled, and thus, the short-term memory of the examinee cannot be effectively tested or dementia patients cannot be properly screened.

In addition, when the number of sentences in the adapted story is greater than 15 or the number of noun-type words is greater than 50, the adapted story is too long and thus difficult to recall, and thus, there is a risk that even a normal person may be misdiagnosed as a dementia patient.

Here, when the number of sentences in the original story is greater than 15, it is preferable that the producer or operator intervenes in the process of generating the adapted story to reduce the number of sentences, or prepares the original story to have 15 sentences or less.

In addition, it is preferable that the adapted story includes at least one additional sentence that is artificially generated to be different from the content of the original story but associated with and connected to the content of the adapted story.

An example of the additional sentence is indicated by A in FIG. 4, and it serves to cause confusion in and thus interruption a recall by the examinee. That is, the examinee, especially a dementia patient, is unable to recall the entire content of the adapted story due to the recall interruption caused by the additional sentence, and as a result, screening of dementia patients is performed more accurately.

Here, that the additional sentence is different from the content of the original story means that the content of the additional sentence is not in the content of the original story, and that the additional sentence is associated with and connected to the content of the adapted story means the content of the additional sentence is connected to the development of the adapted story. The additional sentence may be generated directly by the producer, or automatically generated and produced to include a main character with stimulating content.

In addition, it is preferable that the additional sentence is placed at the end of the adapted story as illustrated in FIG. 4, which is to effectively interrupt a recall of the adapted story by the examinee.

The adapted story completed as described above is converted into voice data and stored in the data storage 100, and is easily output and provided as a voice to the examinee through the voice outputter 200 at a later stage.

The conversion into voice data refers to a process of reading and uttering the adapted story by a person and converting the utterance into digital data by using various digital voice collection equipment and recorders, or a process of directly converting a text into voice data with a mechanical voice by using a text-to-speech conversion device.

In addition, the data storage 100 may store question data and correct answer data for the adapted story.

The question data is data obtained by generating a plurality of questions related to the adapted story by using the changed noun-type words included in the adapted story as correct answers, and converting the questions into voice data, and is used to test the memory of the examinee and provoke utterance.

The correct answer data is data regarding correct answers to the questions, and corresponds to the changed noun-type words included in the adapted story.

It is preferable that the number of questions is 3 to 12, and this is because, when the number of questions is less than 3, the amount of utterance by the examinee may be small and the discrimination performance may be poor, and thus, the memory of the examinee may not be properly tested, and when the number of questions is greater than 12, it is beyond the short-term memory of the examinee, and thus, a memory test may not be performed accurately.

In addition, it is preferable that the questions are about whether an event, an action, a situation, and content presented in the adapted story are correct, and this is to appropriately test the short-term memory through simple and plain fact-oriented questions while easily inducing an utterance of the examinee.

The question may be for requesting an answer in sentences or words, and this is to induce the examinee to answer in short sentences or short answers, so as to secure more voices for dementia diagnosis and extract voice features more accurately.

In addition, the question may be a multiple-choice type in which incorrect answers include noun-type words included in the original story of the same or similar type as the correct answer as illustrated in FIG. 7. For example, in a four-choice question as illustrated in FIG. 7, noun-type words included in the original story, i.e., 'Snow White', 'stepmother', 'seven', 'dwarfs', 'apple', 'horse', and 'prince' are included as incorrect answers among the four choices, causing confusion in a recall by the examinee, such that the examinee selects the content of the fairy tale that the examinee knows, i.e., a wrong answer, and thus, the examinee may be properly identified as a dementia patient.

That is, some examinees cannot recall the content of the adapted story that contains the correct answer to the question, but recall the content of the original story that they have known for a long time, and then select an answer based on the original story, thus, voice features of dementia patients and incorrect answers are induced, and examinees with a lot of voice features of dementia patients and incorrect answers may be diagnosed and screened as dementia patients.

In addition, it is preferable that the last question of the questions is for asking the examinee about his/her correct answer rate. The question for asking the examinee about his/her correct answer rate is indicated by D in FIG. 7 and is for allowing an operator to evaluate the metacognitive ability of the examinee to recognize what he/she knows and does not know. This metacognitive ability assessment may be used as data for confirming once again the accuracy of dementia diagnosis by the present method.

The voice outputter 200 is a unit capable of outputting digital data as a voice, such as a speaker, and serves to output diagnostic voice data stored in the data storage 100 to the outside such that the examinee may listen to the voice.

The examinee, listening to the adapted story of "Snow White" illustrated in FIG. 4 through the voice outputter 200, is confused with the original story of "Snow White" illustrated in FIG. 3 that the examinee already recognizes and remembers, that is, memory interference occurs.

As such, in a state where memory interference occurs, the examinee is requested to recall and utter the content of the adapted story output to the examinee, accordingly, an environment in which the examinee's memory is deteriorated or confusion is caused, and examinees who cannot easily overcome the environment may be quickly and accurately diagnosed and screened as dementia patients.

In addition, as a diagnostic test is performed through voice output of the adapted story, even elderly examinees who are unfamiliar with letters or have difficulty recognizing letters may easily be diagnosed for dementia.

The voice receiver 300 is a unit capable of receiving a voice and converting it into digital data, such as a microphone, and serves to receive a voice spoken by the examinee and convert it into voice data.

That is, voice features of the voice of the examinee received by the voice receiver 300 is analyzed by the controller 400 and then compared with patient voice feature data to be used for determination of dementia.

The controller 400 is connected to the data storage 100, the voice outputter 200, and the voice receiver 300, and controls the data storage 100, the voice outputter 200, and the voice receiver 300 to output story data and then receive a voice of the examinee.

In addition, the controller 400 includes an utterance request unit 410 for requesting the examinee to recall and speak the content of an output story after the output of the story data is completed, a voice feature analysis unit 420 for analyzing voice features in voice data received from the examinee, and a dementia determination unit 430 for determining occurrence of dementia and/or dementia status by comparing the voice features of the examinee analyzed through the voice feature analysis unit 420, with the patient voice feature data.

The controller 400 serves to perform control to analyze the voice features of the examinee, perform dementia determination by using the analyzed voice features, and store, in the data storage 100, dementia information for each examinee determined by the dementia determination unit 430.

The utterance request unit 410 is for requesting the examinee to recall and speak the content of the adapted story, and serves to inform the examinee of the timing of speech to promote and induce an utterance. The utterance request unit 410 outputs utterance request content as a voice, for example, "Recall the content of the story and speak it".

The voice feature analysis unit 420 serves to analyze a voice of the examinee collected through the voice receiver 300 and extract voice features of the examinee. That is, the voice feature analysis unit 420 serves to analyze and extract the fundamental frequency, utterance rate, shimmer, jitter, formant, and voice spectrum of a voice in voice data of the examinee.

The dementia determination unit 430 serves to determine whether the examinee has dementia, by comparing the voice features of the examinee with patient voice feature data, which contains voice features of dementia patients. Here, when the examinee, while recalling and speaking 'Snow White' of the presented adapted story, is confused with the original story of 'Snow White' that the examinee already knows, and exhibits vocal features of dementia patients, the examinee is determined as a dementia patient.

The controller 400 further includes a response request unit 440 for sequentially outputting voice data of questions to the outside after the output of the adapted story to the examinee is completed, to pose the questions to the examinee and request a response. The response request unit 440 serves to check the memory of the examinee through the questions, and induce an utterance through a response.

The controller 400 further includes an auxiliary dementia determination unit 450 for comparing and analyzing responses to the questions collected from the examinee, with correct answers, to identify an incorrect response rate and determine occurrence of dementia and/or dementia status according to the incorrect response rate. The auxiliary dementia determination unit 450 serves to subsidiarily determine dementia based on the incorrect response rate of the responses from the examinee to the questions.

Here, in FIG. 7, correct answers to the questions are indicated by red boxes, other choices than the correct answers are naturally treated as incorrect answers. In FIG. 7, the correct answer to 'Question 8' is confirmed by evaluating 'Question 1' to 'Question 7', and the incorrect answer rate is the proportion of incorrect answers for all questions.

It is preferable that the controller 400 determines whether the examinee has dementia by performing a combination of dementia determination based on the voice features of the examinee and dementia determination based on the incorrect response rate of the examinee, and this is for improving the accuracy of dementia determination.

The controller 400 further includes a recall interruption unit 460 for interrupting a recall by the examinee by allowing a certain period of time to elapse before the output of the adapted story is completed and the examinee is requested to speak. The recall interruption unit 460 interrupts a recall of the adapted story by the examinee through a time delay, to cause the examinee not to well recall the entire content of the adapted story, thereby enabling more accurate screening of dementia patients.

Here, the certain period of time to elapse may be from several seconds to tens of seconds, and during the period of time, a sound such as music may be output to more actively interrupt the recall by the examinee.

The dementia diagnosis system configured as described above may be implemented as an integrated dementia diagnosis system in which the data storage 100, the voice outputter 200, the voice receiver 300, and the controller 400 are modularized and integrated, and may be implemented as an automatic dementia diagnosis device in which all functions are programmed or automated through artificial intelligence or the like.

This dementia diagnosis system further includes a data inputter 500 connected to the data storage 100 and controlled by the controller 400, and a data transmitter 600 connected to the data storage 100 and controlled by the controller 400.

The data inputter 500 is controlled by the controller 400 to input and store various pieces of data, including diagnostic voice data, in the data storage 100. That is, the operator may easily input various pieces of data, including diagnostic voice data, into the present system such that modification, addition, and update of the diagnostic voice data may be appropriately performed.

The data transmitter 600 is, for example, a wired or wireless transmitting/receiving device, and is controlled by the controller 400 to transmit dementia information for each examinee stored in the data storage 100 to an external device through a transmission line or a network.

The data transmitter 600 may transmit dementia information for each examinee, and thus, information about diagnosed and screened dementia patients may be easily provided to management servers of national and private organizations for dementia management and screening.

The dementia diagnosis system configured as described above is a groundbreaking invention that allows quick and accurate screening of dementia by changing key words from an original classic story that an examinee already knows and is familiar with, to new words, presenting a resulting adapted story to cause memory consolidation, and requesting the examinee to recall and speak the adapted story, so as to maximize a memory interference effect between the adapted story and the original classic story.

A dementia diagnosis system using story-based voice extraction includes: a data storage 10 for storing diagnostic voice data including story data for dementia diagnosis, patient voice feature data extracted by comparing and analyzing voice features of normal people with voice features of dementia patients, and dementia information for each examinee; a voice output device 20 configured to output the diagnostic voice data stored in the data storage 10 to the outside to allow the examinee to listen to the diagnostic voice data; a voice receiver 30 configured to receive a voice spoken by the examinee and convert the voice into voice data; and a controller 40 that is connected to the data storage 10, the voice outputter 20, and the voice receiver 30, is configured to control the voice receiver 30, the data storage 10, and the voice outputter 20 to receive a voice of the examinee after outputting the story data, and includes an utterance request unit 41 configured to request the examinee to recall and speak the content of an output story after the output of the story data is completed, a voice feature analysis unit 42 configured to analyze voice features in voice data received from the examinee, and a dementia determination unit 43 configured to determine occurrence of dementia and/or dementia status by comparing the voice features of the examinee analyzed through the voice feature analysis unit 42, with the patient voice feature data, the controller 40 being further configured to perform control to store, in the data storage 10, dementia information for each examinee determined by the dementia determination unit 43, wherein the story data is obtained by generating an original story that includes a plurality of sentences and is in the form of a story centered around a character that the examinee already knows and an event related to the character, extracting some of noun-type words included in the original story, changing the extracted noun-type words to other noun-type words of the same or similar type to generate an adapted story, and converting the adapted story into voice data.

In the dementia diagnosis system using story-based voice extraction, the data storage 10 stores question data and correct answer data for the adapted story, the question data is data obtained by generating a plurality of questions related to the adapted story by using the changed noun-type words included in the adapted story as correct answers, and converting the questions into voice data, and the controller 40 further includes a response request unit 44 configured to sequentially output voice data of questions to the outside after the output of the adapted story to the examinee is completed, to pose the questions to the examinee and request a response.

In the dementia diagnosis system using story-based voice extraction, the questions include 3 to 12 questions, and are questions for requesting responses about whether events, actions, situations, and content presented in the adapted story are correct.

In the dementia diagnosis system using story-based voice extraction, the questions are for requesting responses in sentences or words.

In the dementia diagnosis system using story-based voice extraction, the questions are of a multiple-choice type in which incorrect answers include noun-type words included in the original story of the same or similar type as correct answers, and a question for asking the examinee about his/her correct answer rate may be placed as the last question.

In the dementia diagnosis system using story-based voice extraction, the controller 40 further includes an auxiliary dementia determination unit 45 configured to compare and analyze responses to the questions collected from the examinee, with correct answers, to identify an incorrect response rate and determine occurrence of dementia and/or dementia status according to the incorrect response rate.

In the dementia diagnosis system using story-based voice extraction, the controller 40 determines whether the examinee has dementia by performing a combination of dementia determination based on the voice features of the examinee and dementia determination based on the incorrect response rate of the examinee.

In the dementia diagnosis system using story-based voice extraction, the original story is a fairy tale or a fable that the examinee already knows.

In the dementia diagnosis system using story-based voice extraction, the adapted story consists of 5 to 15 sentences and contains 20 to 50 different noun-type words.

In the dementia diagnosis system using story-based voice extraction, the adapted story includes at least one artificially generated additional sentence, which is different from content of the original story, is associated with and connected to content of the adapted story, and is placed at an end of the adapted story.

In the dementia diagnosis system using story-based voice extraction, the controller 40 further includes a recall interruption unit 46 configured to interrupt a recall by the examinee by allowing a certain period of time to elapse before the output of the adapted story is completed and the examinee is requested to speak.

In the dementia diagnosis system using story-based voice extraction, the voice features include one or more of the fundamental frequency, utterance rate, pause, shimmer, jitter, formant, and voice spectrum of a voice.

The dementia diagnosis system using a story-based question-and-answer-type assessment further includes: a data inputter 50 connected to the data storage 10, controlled by the controller 40, and configured to input and store various pieces of data, including diagnostic voice data, into the data storage (10); and a data transmitter 60 connected to the data storage 10, controlled by the controller 40, and configured to transmit dementia information for each examinee stored in the data storage 10 to an external device.

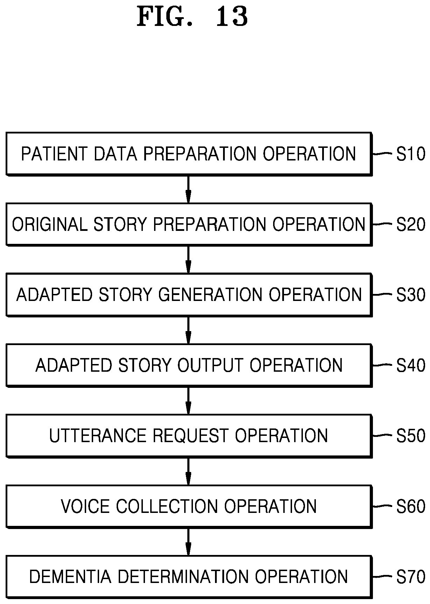

FIG. 13 is a flowchart of a dementia diagnosis method through story-based voice extraction, according to the present disclosure.

Referring to FIG. 13, a dementia diagnosis method using story-based voice extraction according to the present disclosure includes a patient data preparation operation (S10) of extracting voice features of dementia patients and preparing the voice features as patient voice feature data, an original story preparation operation (S20) of preparing an original story, an adapted story generation operation (S30) of preparing an adapted story by adapting the original story, and converting the adapted story into voice data, an adapted story output operation (S40) of outputting the voice data of the adapted story to the outside, an utterance request operation (S50) of requesting an examinee to speak the content of the adapted story, a voice collection operation (S60) of receiving and collecting a voice spoken by the examinee, a dementia determination operation (S70) of determining dementia by comparing the collected voice of the examinee with the patient voice feature data, and these operations are sequentially performed to diagnose dementia.

The dementia diagnosis method may further include, after the adapted story generation operation (S30), a question generation operation (S30a) of preparing a plurality of questions related to the adapted story, with changed noun-type words included in the adapted story as correct answers, and converting the plurality of questions into voice data (S30a), and the utterance request operation (S50) may further include a question output process of sequentially outputting the voice data of the questions to the outside to pose the questions to the examinee, after the output of the adapted story to the examinee is completed.

In the question generation operation (S30a), the questions may include 3 to 12 questions, and may be for requesting responses regarding whether events, actions, situations, and content presented in the adapted story are correct.

The question may be for requesting a response in sentences or words.

The question may be of a multiple-choice type in which incorrect answers include noun-type words included in the original story of the same or similar type as correct answers, and a question for asking the examinee about his/her correct answer rate may be placed as the last question.

The dementia determination operation (S70) further includes an auxiliary dementia determination process of comparing and analyzing responses to the questions collected from the examinee, with correct answers, to identify an incorrect response rate and determine occurrence of dementia and/or dementia status according to the incorrect response rate.

The dementia determination operation (S70) may determine whether the examinee has dementia by performing a combination of dementia determination based on the voice features of the examinee and dementia determination based on the incorrect response rate of the examinee.

In the original story preparation operation (S20), the original story may be a fairy tale or a fable that the examinee already knows.

In the adapted story generation operation (S30), the adapted story may consist of 5 to 15 sentences and contain 20 to 50 different noun-type words.

The adapted story generation operation (S30) further includes a sentence addition process of adding at least one artificially generated additional sentence that is different from the content of the original story and is associated with and connected to the content of the adapted story, and the additional sentence may use story-based voice extraction that is placed at the end of the adapted story.

The utterance request operation (S50) may further include a recall interruption process of interrupting a recall by the examinee by allowing a certain period of time to elapse before the output of the adapted story is completed and the examinee is requested to speak.

In the patient data preparation operation (S10), the voice features may be one or more of the fundamental frequency, utterance rate, pause, shimmer, jitter, formant, and voice spectrum of a voice.

An embodiment of the present disclosure may be implemented as a computer program that may be executed through various components on a computer, and such a computer program may be recorded in a computer-readable medium. In this case, the medium may include a magnetic medium, such as a hard disk, a floppy disk, or a magnetic tape, an optical recording medium, such as a compact disc read-only memory (CD-ROM) or a digital video disc (DVD), a magneto-optical medium, such as a floptical disk, and a hardware device specially configured to store and execute program instructions, such as ROM, random-access memory (RAM), or flash memory.

Meanwhile, the computer program may be specially designed and configured for the present disclosure or may be well-known to and usable by those skilled in the art of computer software. Examples of the computer program may include not only machine code, such as code made by a compiler, but also high-level language code that is executable by a computer by using an interpreter or the like.

According to an embodiment, the method according to various embodiments of the present disclosure may be included in a computer program product and provided. The computer program product may be traded as commodities between sellers and buyers. The computer program product may be distributed in the form of a machine-readable storage medium (e.g., a CD-ROM), or may be distributed online (e.g., downloaded or uploaded) through an application store (e.g., Play Store^{™}) or directly between two user devices. In a case of online distribution, at least a portion of the computer program product may be temporarily stored in a machine-readable storage medium such as a manufacturer's server, an application store's server, or a memory of a relay server.

The operations of the methods according to the present disclosure may be performed in any suitable order unless otherwise indicated herein or otherwise clearly contradicted by context. The present disclosure is not limited to the described order of the operations. The use of any and all examples, or exemplary language (e.g., 'and the like') provided herein, is intended merely to better illuminate the present disclosure and does not pose a limitation on the scope of the present disclosure unless otherwise claimed. Also, numerous modifications and adaptations will be readily apparent to those skill in the art without departing from the spirit and scope of the present disclosure.

Although specific embodiments have been described in the detailed description of the present disclosure, it will be apparent to those skilled in the art that various modifications are possible without departing from the scope of the present disclosure.

## Claims

1. A dementia diagnosis system using a story-based question-and-answer assessment, the dementia diagnosis system comprising:
a data inputter (10) configured to input text data regarding an original story;
a data storage (20) configured to store the text data that is input through the data inputter (10), and dementia information for each examinee;
a voice outputter (30) configured to output diagnostic voice data to an outside to allow an examinee to listen to the diagnostic voice data;
a voice receiver (40) configured to receive a response spoken by the examinee; and
a controller (50) that is connected to the data inputter (10), the data storage (20), the voice outputter (30), and the voice receiver (40), and comprises a story data generation unit (51) configured to automatically generate adapted story data, question data, and correct answer data based on an original story that is input as text data, a response data generation unit (52) configured to receive a response from the examinee and generate response data, a dementia determination unit (53) configured to compare and analyze the correct answer data with the response data to identify an incorrect response rate, and determine occurrence of dementia and/or dementia status according to the incorrect response rate, the controller (50) being configured to control the data storage (20), the voice outputter (30), and the voice receiver (40) to sequentially output the adapted story data and the question data, receive responses from the examinee to output questions, and store identified dementia information for each examinee,
wherein the original story is in a form of a story centered around a character that the examinee already knows, and an event related to the character, and consists of a plurality of sentences,
the adapted story data is data obtained by extracting some of noun-type words included in the original story, changing the extracted noun-type words to other noun-type words of a same or similar type to generate an adapted story, and converting the adapted story into voice data,
the question data is data obtained by generating a plurality of questions related to the adapted story by using the changed noun-type words included in the adapted story as correct answers, and converting the questions into voice data,
the correct answer data is data obtained by extracting the correct answers corresponding to the questions and converting the extracted correct answers into voice data, and
the response data is voice data obtained by receiving and storing responses from the examinee to the questions through the voice receiver (40).

2. The dementia diagnosis system of claim 1, wherein the questions comprise 3 to 12 questions, are about whether events, actions, situations, and content presented in the adapted story are correct, and are of a multiple-choice type in which incorrect answers comprise noun-type words included in the original story of a same or similar type as correct answers, and a question for asking the examinee about a correct answer rate of the examinee is placed as a last question.

3. The dementia diagnosis system of claim 1, wherein the original story is a fairy tale or a fable that the examinee already knows.

4. The dementia diagnosis system of claim 1, wherein the adapted story consists of 5 to 15 sentences, contains 20 to 50 different noun-type words, and comprises at least one artificially generated additional sentence, which is different from content of the original story, is associated with and connected to content of the adapted story, and is placed at an end of the adapted story.

5. The dementia diagnosis system of claim 1, wherein the controller (50) is further configured to receive a story notification text for notifying that the adapted story is to be output and then a question related to the adapted story is to be presented, convert the story notification text into voice data, and control the voice outputter (30) to output the story notification text before outputting the adapted story.

6. The dementia diagnosis system of claim 5, wherein the adapted story is a fairy tale or a fable that the examinee already knows, and
a sentence containing a title of the fairy tale or fable presented as the adapted story is placed at beginning of the story notification text.

7. The dementia diagnosis system of claim 1, wherein the controller (50) is further configured to receive a question notification text for notifying that a preset number of questions are to be presented, convert the question notification text into voice data, and control the voice outputter (30) to output the question notification text after the output of the adapted story is completed.

8. The dementia diagnosis system of claim 1, wherein the dementia determination unit (53) is further configured to determine, based on the incorrect response rate being greater than at least 25 %, that the examinee has dementia, determine, based on the incorrect response rate being 25 % to 40 %, that the examinee has mild dementia, and determine, based on the incorrect response rate being greater than at least 40 %, that the examinee has severe dementia.

9. The dementia diagnosis system of claim 1, wherein a plurality of original stories are prepared and input to generate a plurality of adapted stories, respectively, and questions and correct answers related to each of the adapted stories are generated, and
the controller (50) further comprises a known story identification unit (54) configured to, before the adapted story is output, output a story identification text as a voice to the examinee for identifying which story the examinee already knows from among a plurality of adapted stories, and a story selection unit (55) configured to select an adapted story that is identified to be already known by the examinee such that the adapted story identified through the known story identification unit (54) is output.

10. The dementia diagnosis system of claim 1, wherein a plurality of original stories are prepared and input to generate a plurality of adapted stories, respectively, and questions and correct answers related to each of the adapted stories are generated, and
the controller (50) further comprises a used story identification unit (56) configured to output a voice to the examinee before the adapted story is output, to identify which story among the plurality of adapted stories has been previously used for a diagnostic test, and a story exclusion unit (57) configured to exclude, from output, an adapted story identified by the used story identification unit (56) to have been previously used.

11. The dementia diagnosis system of claim 1, further comprising:
a display (60) connected to the controller (50) and configured to display text data on a screen; and
a text inputter (70) connected to the controller (50) and configured to modify the text data by inputting a text,
wherein the controller (50) further comprises a story confirmation/modification unit (58) configured to display the adapted story as text data on the display (60) to allow a producer or an operator to confirm the adapted story and artificially modify the adapted story through the text inputter (70).

12. The dementia diagnosis system of claim 1, further comprising:
a display (60) connected to the controller (50) and configured to display text data on a screen; and
a text inputter (70) connected to the controller (50) and configured to modify the text data by inputting a text,
wherein the controller (50) further comprises a question confirmation/modification unit (59) configured to display the questions as text data on the display (60) to allow a producer or an operator to confirm the questions and artificially modify the questions through the text inputter (70).

13. The dementia diagnosis system of claim 1, further comprising a data transmitter (80) that is connected to the data storage (20), controlled by the controller (50), and configured to transmit the dementia information for each examinee stored in the data storage (20) to an external device.

14. A dementia diagnosis method using a story-based question-and-answer assessment, the dementia diagnosis method comprising:
an original story preparation operation (S1) of preparing an original story that is in a form of a story centered around a character that an examinee already knows, and an event related to the character, and consists of a plurality of sentences;
an original story input operation (S2) of converting the prepared original story into text data and inputting the text data into a diagnostic device;
an adapted story generation operation (S3) of extracting some of noun-type words included in the input original story and changing the extracted noun-type words to other noun-type words of a same or similar type to generate an adapted story in a form of text data;
a question generation operation (S4) of generating a plurality of questions related to the adapted story in a form of text data by using the changed noun-type words included in the adapted story as correct answers;
a voice data conversion operation (S5) of converting the text data of the adapted story and the text data of the questions into voice data;
an adapted story output operation (S6) of outputting the audio data of the adapted story to an outside to provide the audio data to the examinee;
a question output operation (S7) of, after the outputting of the adapted story to the examinee is completed, sequentially outputting the voice data of the questions to the outside to pose the questions to the examinee;
a response reception operation (S8) of receiving responses from the examinee to the questions, respectively; and
a dementia determination operation (S9) of comparing and analyzing the received responses and the correct answers to identify an incorrect response rate, and determining occurrence of dementia and/or dementia status according to the incorrect response rate.

15. A computer-readable recording medium having recorded thereon a program for causing a computer to execute the method of claim 14.
